# FASCICULE DE BREVET EUROPEEN

(11) **EP 3 831 443 B1**
(45) Date de publication et mention de la délivrance du brevet: **16.08.2023**
(21) Numéro de dépôt: 20211456.7
(22) Date de dépôt: 03.12.2020
(51) Int. Cl.: A61N 1/365, A61B 5/0535, A61B 5/0538, A61B 5/02, A61B 5/287, A61B 5/352, A61N 1/362, A61N 1/375, A61N 1/39, A61B 5/00

(54) **SYSTÈME MÉDICAL IMPLANTABLE POUR MESURER UN PARAMÈTRE PHYSIOLOGIQUE**
IMPLANTIERBARES MEDIZINPRODUKT ZUM MESSEN EINES PHYSIOLOGISCHEN PARAMETERS
IMPLANTABLE MEDICAL SYSTEM FOR MEASURING A PHYSIOLOGICAL PARAMETER

(30) Priorité: 03.12.2019 FR 1913677
(43) Date de publication de la demande: 09.06.2021
(73) Titulaire: Sorin CRM SAS, 92140 Clamart (FR)
(72) Inventeur: MALDARI, Mirko, 75019 Paris (FR)
(74) Mandataire: Ungerer, Olaf

(56) Documents cités:
- US-A- 4 686 987
- US-A1- 2007 055 170
- US-A1- 2013 138 008
- US-A1- 2018 021 585

## Description

La présente invention se rapporte à un système médical implantable configuré pour réaliser une mesure d'un paramètre physiologique.

La cardiographie par impédance est une technique connue pour mesurer des paramètres physiologiques, en particulier des paramètres hémodynamiques.

La cardiographie par impédance peut être utilisée pour des mesures non-invasives au moyen d'un dispositif externe couplé à des électrodes de surface.

Dans le cas d'un système médical implantable, il est connu que les mesures de cardiographie par impédance soient effectuées par injection de courant et par une mesure de la tension entre deux électrodes d'une même sonde. Le document de l'art antérieur US 2019/0111268 A se rapporte à une telle mesure d'impédance locale entre deux électrodes d'une même sonde sous cutanée. Le document US 2019/0111268 A se propose de détecter un trouble du rythme cardiaque, précisément une arythmie cardiaque, sur la base d'événements cardiaques identifiés à partir d'un signal électrique.

Il est cependant connu de distinguer les troubles du rythme cardiaque, comme l'arythmie cardiaque, correspondant à une variation anormale du rythme des battements du coeur perturbant son bon fonctionnement, d'une insuffisance cardiaque qui est une incapacité du coeur à pomper une quantité de sang suffisante pour assurer un débit sanguin satisfaisant au niveau de l'ensemble du corps.

L'insuffisance cardiaque peut ne toucher qu'une partie du coeur, ou l'ensemble de celui-ci. L'insuffisance cardiaque a une évolution chronique et progressive, généralement lente, qui peut se compter en années.

Il s'avère que la détection d'événements cardiaques telle que décrite dans US 2019/0111268 A pour identifier un trouble du rythme cardiaque n'est pas adaptée à la surveillance d'insuffisance cardiaque. En effet, la mesure d'impédance proposée dans le document US 2019/0111268 A est relative à une mesure locale, entre deux électrodes d'une même sonde, ce qui la rend peu sensible à l'activité pulmonaire et à la circulation sanguine dans les organes voisins. Or les informations respiratoires et hémodynamiques relatives à l'activité pulmonaire et à la circulation sanguine dans les organes voisins sont autant d'informations utiles au diagnostic et à la surveillance d'une insuffisance cardiaque.

Les documents US 2007/055170 A1 et US2013138008 A1 montrent chacun un dispositif médical implantable configuré pour associer un signal d'impédance et un signal d'électrocardiogramme pour en déterminer un paramètre représentatif d'une période pré-éjectionnelle.

Le document US 4 686 987 A montre un dispositif médical implantable de mesure d'impédance basé sur une détection d'enveloppe.

Le document US 2018/021585 A1 montre un système comprenant deux dispositifs médicaux implantables configurées pour une mesure d'impédance.

Ainsi, l'objet de la présente invention est d'améliorer et d'optimiser le diagnostic et la surveillance d'une insuffisance cardiaque.

L'objet de la présente invention est atteint avec un système médical implantable pour mesurer au moins un paramètre physiologique tel que défini par la revendication 1. Des modes de réalisations de l'invention sont définis par les revendications dépendantes.

Le système médical implantable comprend au moins un dipôle émetteur formé de deux électrodes connectées à un générateur et configuré pour émettre un signal électrique ; au moins un dipôle récepteur formé de deux électrodes, chacune distincte des électrodes du dipôle émetteur, le dipôle récepteur étant configuré pour recueillir le signal électrique émis au moyen du dipôle émetteur ; et un module d'analyse comprenant au moins un amplificateur, un détecteur d'enveloppe et un convertisseur analogique-numérique et un moyen de traitement pour traiter le signal électrique recueilli au moyen du dipôle récepteur ; et un moyen configuré pour réaliser un électrocardiogramme ou un électrogramme ; le module d'analyse étant en outre configuré pour associer le signal électrique traité et l'électrocardiogramme pour en déterminer un paramètre représentatif d'une période pré-éjectionnelle.

Le fait que le système ait au moins quatre électrodes, de sorte que le dipôle émetteur soit distinct du dipôle récepteur, permet d'obtenir une mesure d'impédance plus globale et donc davantage représentative du milieu environnant, en particulier plus globale et plus représentative du milieu environnant qu'une mesure entre seulement deux électrodes d'une même sonde. En effet, le présent système permet de récupérer des informations mécaniques physiologiques au moyen des deux dipôles en analysant le signal électrique reçu et traité, dont l'amplitude a été modulée selon les propriétés électriques du milieu de propagation entre le dipôle émetteur et le dipôle récepteur.

Ainsi, un paramètre représentatif d'une période pré-éjectionnelle peut être extrait de l'atténuation de la tension entre le dipôle émetteur et le dipôle récepteur en tenant compte de l'électrocardiogramme ou de l'électrogramme. La détermination du paramètre représentatif d'une période pré-éjectionnelle fournit un indicateur adapté au diagnostic et à la surveillance de l'insuffisance cardiaque.

La présente invention peut être davantage améliorée grâce aux modes de réalisation suivants. Selon un mode de réalisation, le module d'analyse peut être configuré pour extraire du signal électrique traité une variation de volume et/ou une variation de pression en fonction du temps proportionnelle(s) à une baisse de tension entre le dipôle émetteur et le dipôle récepteur.

La détermination de la variation de volume du coeur et des poumons permet avantageusement de récupérer des informations hémodynamiques et respiratoires à partir du même signal électrique traité. La détermination de la variation de volume du coeur peut permettre d'identifier l'ouverture de la valvule aortique.

Selon un mode de réalisation, la détermination de la période pré-éjectionnelle peut comprendre la détection de l'onde R ou de l'onde Q d'un complexe QRS recueilli à partir du moyen de détection.

Le temps du début de l'onde Q ou de l'onde R est un paramètre nécessaire pour pouvoir déterminer la période pré-éjectionnelle.

En considérant un électrocardiogramme, le temps du début de l'onde Q peut être identifié à partir de l'onde R du complexe QRS car l'onde R est davantage dominante que l'onde Q (le pic R ayant une amplitude plus marquante que celle du pic Q) et donc plus facilement détectable que l'onde Q.

La détection de l'onde R peut également être utilisée en tant que telle comme indicatrice du temps de début de la période pré-éjectionnelle et ainsi permettre de réduire la complexité du module d'analyse du système nécessaire à l'identification de l'onde Q. La détection de l'onde R peut être réalisée à partir d'un électrocardiogramme ou d'un électrogramme.

Selon un mode de réalisation, le module d'analyse peut être apte à surveiller en outre un paramètre représentatif de l'efficacité d'une thérapie en tenant compte du paramètre représentatif d'une période pré-éjectionnelle.

Le module d'analyse du présent système est ainsi apte à évaluer un paramètre hémodynamique tel que la fraction d'éjection ou le volume d'éjection, qui sont des paramètres permettant d'évaluer la performance cardiaque. Le présent système est ainsi davantage adapté pour la surveillance d'une insuffisance cardiaque et de la thérapie prescrite.

Selon un mode de réalisation, les deux électrodes du dipôle récepteur peuvent être configurées pour recueillir simultanément le signal électrique émis et un électrocardiogramme ou un électrogramme.

Ainsi, les mêmes électrodes peuvent à la fois servir d'électrodes de détection et de dipôle récepteur. Le dipôle récepteur a ainsi une double fonction, ce qui permet d'optimiser le système en réduisant le nombre d'électrodes nécessaires.

Selon un mode de réalisation, l'activation du module d'analyse peut être déclenchée par la détection d'au moins un pic d'un complexe PQRST recueilli à partir du moyen de détection (26). Ainsi, la consommation énergétique du système peut être réduite en limitant la période durant laquelle le module d'analyse du système est activé. De manière avantageuse, le module d'analyse peut par exemple être activé durant une fenêtre de temps qui peut commencer à partir de la détection d'au moins un pic du complexe PQRST.

Selon un mode de réalisation, le système peut comprendre un moyen apte à détecter une activité mécanique du coeur à partir d'un signal acoustique représentatif des sons du coeur et dans lequel le module d'analyse peut être configuré pour comparer ledit signal acoustique au signal électrique traité.

Le signal acoustique permet de révéler l'activité mécanique des valves cardiaques. L'analyse des différents types de signaux permet de mieux les caractériser et de faire ressortir les informations utiles pour la surveillance d'une insuffisance cardiaque.

De ce fait, le système est configuré pour détecter et recueillir l'activité acoustique du coeur. Les informations ainsi recueillies relatives à l'activité acoustique peuvent être utilisées pour corréler le signal électrique traité et recueilli par le module d'analyse du système.

Selon l'invention, le système peut comprendre un premier dispositif médical implantable pourvu du dipôle émetteur, et un deuxième dispositif médical implantable distinct du premier dispositif médical implantable et pourvu du dipôle récepteur.

Ainsi, il est possible d'obtenir une mesure d'impédance plus globale et donc davantage représentative du milieu environnant, en particulier plus globale et plus représentative du milieu environnant qu'une mesure entre seulement deux électrodes d'une même sonde. En effet, les dipôles sont couplés galvaniquement entre eux et un champ électrique se propage à travers le corps humain du dipôle émetteur jusqu'au dipôle récepteur. Le signal électrique reçu par le dipôle récepteur est alors modulé en amplitude. En démodulant l'amplitude du signal reçu, il est possible de récupérer au moins un paramètre physiologique à partir duquel un paramètre représentatif d'une période pré-éjectionnelle peut être défini.

Selon un mode de réalisation, l'un du premier dispositif médical implantable ou du deuxième dispositif médical implantable peut être un défibrillateur automatique implantable sous-cutané ou un enregistreur d'événement ; et l'autre du premier dispositif médical implantable ou du deuxième dispositif médical implantable peut être un dispositif endocavitaire implantable.

Ainsi, le champ électrique émis par le dipôle émetteur se propage à travers un canal permettant à la fois de récupérer des informations hémodynamiques et respiratoires. Le présent système médical implantable pour mesurer au moins un paramètre physiologique est ainsi configuré pour utiliser des dispositifs médicaux implantable préexistants, c'est-à-dire des dispositifs qui ont une fonction supplémentaire en plus de la détermination d'un paramètre représentatif d'une période pré-éjectionnelle, comme une fonction de défibrillation.

Selon un mode de réalisation, le dispositif endocavitaire implantable peut être un stimulateur cardiaque sans sonde.

Un stimulateur cardiaque sans sonde permet une procédure d'implantation moins invasive que les dispositifs implantables avec sonde. Ainsi, un stimulateur cardiaque sans sonde permet d'éviter les complications liées aux sondes transveineuses et au générateur d'impulsions sous-cutané utilisés dans les stimulateurs cardiaques classiques.

L'invention et ses avantages seront expliqués plus en détail dans ce qui suit au moyen de modes de réalisation préférés et en s'appuyant notamment sur les figures d'accompagnement suivantes, dans lesquelles :
La Figure 1 représente une vue schématisée d'un système médical implantable
La Figure 2 représente une vue schématisée de la propagation d'un signal électrique entre un dipôle émetteur et un dipôle récepteur ;
La Figure 3 représente l'intégration du système médical implantable dans un dispositif implantable sous cutané;
La Figure 4 représente l'intégration du système médical implantable selon la présente invention dans un système de multi-dispositifs ;
La Figure 5 représente un électrocardiogramme et un signal électrique traité obtenu par le système médical implantable selon la présente invention intégré dans le système de multi-dispositifs de la Figure 4 ;
La Figure 6 montre une corrélation entre le signal électrique traité obtenu par le système médical implantable selon la présente invention intégré dans le système de multi-dispositifs de

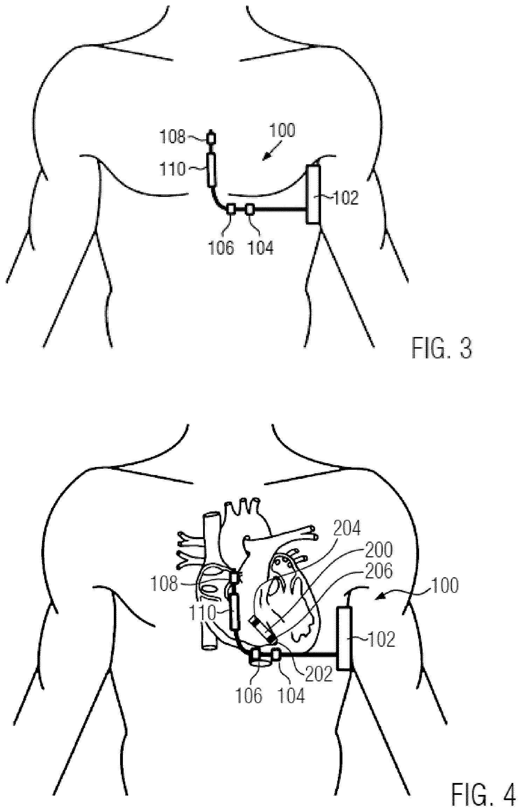

la Figure 4 et des courbes représentant des variations de pression du ventricule gauche et de l'aorte ;

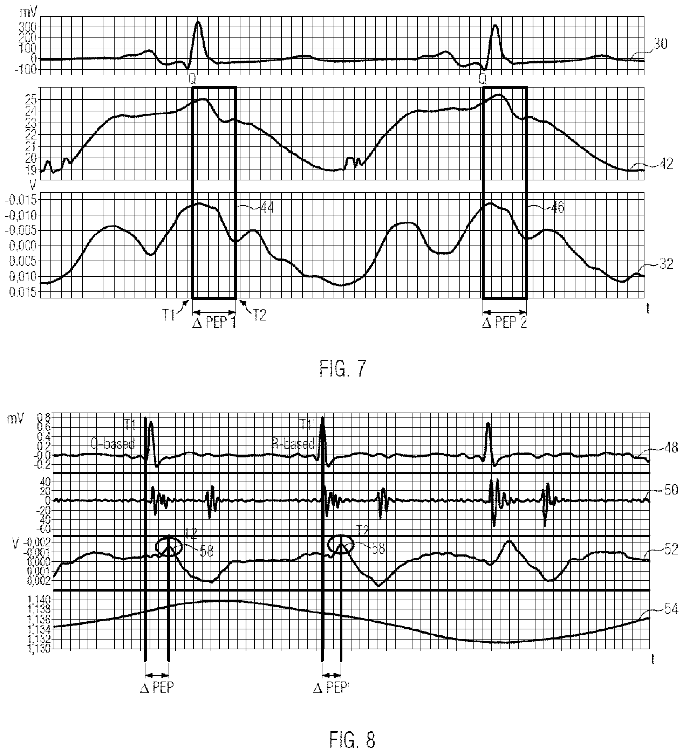

La Figure 7 montre une corrélation entre le signal électrique traité obtenu par le système médical implantable selon la présente invention intégré dans le système de multi-dispositifs de la Figure 4 et une courbe représentant des variations de volume du ventricule gauche ;
La Figure 8 représente un électrocardiogramme, un phonocardiogramme, et des signaux électriques obtenus par le système médical implantable intégré dans le dispositif implantable sous cutanée de la Figure 3.

L'invention va maintenant être décrite plus en détail en utilisant des modes de réalisation avantageux d'une manière exemplaire et en référence aux figures. Les modes de réalisation décrits sont simplement des configurations possibles et il faut garder à l'esprit que les caractéristiques individuelles telles que décrites ci-dessus peuvent être fournies indépendamment les unes des autres ou peuvent être omises tout à fait lors de la mise en oeuvre de la présente invention.

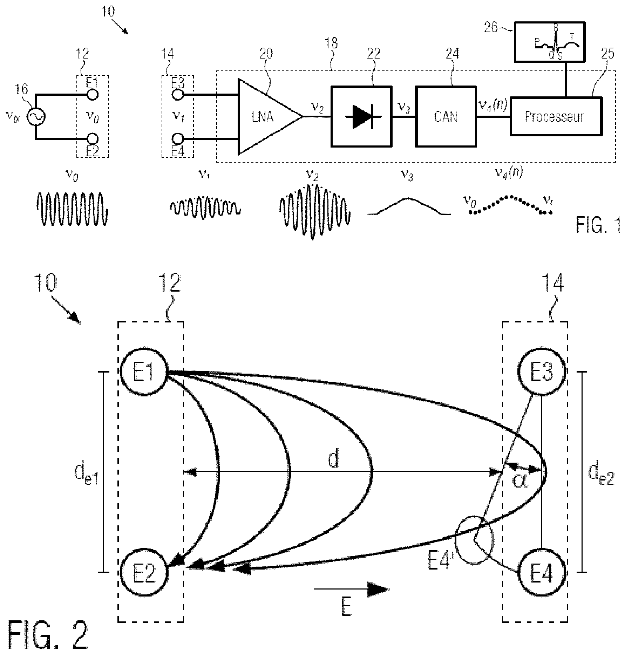

La **Figure 1** illustre un exemple de médical implantable 10. De plus, la partie inférieure de la Figure 1 illustre des signaux électriques V₀ à V₄.

Le système médical implantable 10 comprend un dipôle émetteur 12 et un dipôle récepteur 14. Le dipôle émetteur 12 est formé par un couple d'électrodes E1, E2. Le dipôle récepteur 14 est formé par un couple d'électrodes E3, E4. Les électrodes E1, E2 sont distinctes des électrodes E3, E4.

Les deux électrodes E1, E2 du dipôle émetteur 12 sont connectées à un générateur 16 qui est configuré pour générer un signal électrique d'entrée Va à une fréquence définie fo. Notons que la fréquence f₀ doit être suffisamment élevée pour ne pas stimuler le coeur du patient en interférant avec l'activité cardiaque normale du patient. En particulier, la fréquence définie f₀ est supérieure à 1kHz, en particulier, elle peut être supérieure à 10kHz. Le générateur 16 peut être un générateur de tension ou de courant.

Le système médical implantable 10 comprend également un module d'analyse 18. Le module d'analyse 18 comprend un amplificateur 20, en particulier un amplificateur passe bande à faible bruit qui permet d'amplifier le signal V₁ à la fréquence fo.

L'amplificateur 20 peut comprendre un filtre analogique.

Dans une variante, l'amplificateur 20 peut être constitué d'une pluralité d'amplificateurs à faible bruit. Dans cette variante, la sélection d'un des amplificateurs à faible bruit est effectuée en fonction de la position mutuelle du dipôle émetteur 12 et du dipôle récepteur 14 qui affecte l'atténuation du signal électrique. De cette façon, la consommation énergétique du système 10 peut être optimisée en activant seulement l'amplificateur à faible bruit nécessaire pour avoir une détection du signal électrique suffisante pour la mesure, c'est-à-dire qui respecte un certain rapport signal sur bruit prédéfini.

Dans une autre variante, l'amplificateur 20 peut être un amplificateur à gain variable et/ou un amplificateur à gain programmable.

Le module d'analyse 18 comprend en outre un détecteur d'enveloppe 22 et un convertisseur analogique-numérique 24 qui sont configurés pour traiter un signal électrique recueilli au moyen du dipôle récepteur 14.

Le module d'analyse 18 comprend de plus un moyen de traitement 25. Le moyen de traitement 25 est un processeur 25. Le processeur 25 peut comprendre un moyen de filtrage numérique afin de traiter numériquement le signal électrique V₄. Dans une variante, le moyen de filtrage numérique est arrangé entre le convertisseur analogique-numérique 24 et le processeur 25.

Le processeur 25 est connecté à un moyen de détection 26 configuré pour réaliser un électrocardiogramme ou un électrogramme.

Dans un exemple où le moyen de détection 26 est un dispositif sous-cutané, tel qu'un défibrillateur implantable sous-cutané ou un enregistreur implantable en boucle, le moyen de détection 26 est configuré pour réaliser un électrocardiogramme, en particulier un électrocardiogramme sous-cutané. Ainsi, le moyen de détection 26 est apte à détecter un complexe PQRST, et en particulier les ondes P, Q et R.

Dans un autre exemple où le moyen de détection 26 est un dispositif implantable endocavitaire, tel qu'un stimulateur cardiaque implantable sans sonde en forme de capsule, le moyen de détection 26 est configuré pour un réaliser un électrogramme. Ainsi, le moyen de détection 26 est apte à détecter l'onde R à partir d'une mesure locale de la dépolarisation du ventricule droit. L'activation du module d'analyse 18 peut être déclenchée à partir des signaux du module de détection 26, notamment à partir de la détection de l'onde R afin d'optimiser la durée de fonctionnement des composants actifs du module d'analyse 18 et donc de réduire la consommation énergétique requise par le système 10.

Afin de réduire davantage la consommation énergétique du système 10, la mesure du paramètre représentatif d'une période pré-éjectionnelle peut être réalisée seulement pendant une fenêtre de temps prédéterminée à intervalle régulier. La fréquence des intervalles de mesure peut également être réduite afin de faire des économies d'énergie.

Les deux électrodes E3, E4 du dipôle récepteur 14 peuvent être configurées pour recueillir simultanément le signal électrique.

Dans une variante, les deux électrodes E3, E4 du dipôle récepteur 14 sont configurées pour recueillir simultanément le signal électrique émis et l'électrocardiogramme.

Le processeur 25 du module d'analyse 18 est en outre configuré pour associer le signal électrique traité et l'électrocardiogramme pour en déterminer un paramètre représentatif d'une période pré-éjectionnelle.

Le fonctionnement du système médical implantable 10 est davantage expliqué dans ce qui suit en utilisant les signaux électriques V₀ à V₄ illustrés à la Figure 1. La Figure 1 illustre un signal électrique d'entrée V₀ avec une amplitude fixe. Dans une variante, le signal électrique d'entrée V₀ peut avoir une amplitude variable afin d'améliorer le rapport signal sur bruit.

Dans une autre variante, l'amplitude du signal électrique d'entrée V₀ peut être ajustée à partir du feed-back du dipôle récepteur 14 qui, dans cette variante, est en outre configuré pour une transmission par télémétrie avec un dispositif tiers, par exemple un dispositif externe.

Le dipôle récepteur 14 recueille un signal électrique V₁ différent de V₀ car le champ électrique s'est propagé, suite à l'application du signal électrique Vo, à travers un volume, également appelé canal, par exemple des tissus humains, dont l'impédance est non nulle.

Le signal électrique V₁ est ensuite amplifié au moyen de l'amplificateur 20 résultant en un signal électrique amplifié V₂. L'enveloppe, représentée par le signal V₃, du signal électrique V₂ est déterminée au moyen du détecteur d'enveloppe 22, en particulier par une démodulation en amplitude du signal électrique V₂. L'enveloppe du signal électrique V₃ est ensuite échantillonnée par le convertisseur analogique-numérique 24 de manière à obtenir un signal numérique V₄(n) où n représente le nombre d'échantillons.

Le signal numérique V₄ peut être traité au moyen du processeur 25 et davantage filtré numériquement afin de discriminer les informations respiratoires des informations hémodynamiques récupérées à partir du signal électrique traité.

Notons que la fréquence de coupure des filtres peut être ajustée en fonction des caractéristiques de chaque paramètre physiologique à déterminer. Par exemple, un filtre passe bas avec une fréquence de coupure f_{c} comprise entre f_{c}=0,5Hz et 5Hz, en particulier f_{c}=1Hz, est utilisé pour isoler des signaux respiratoires tandis qu'un filtre passe-bande avec f_{c1}= 1Hz et f_{c2}=30Hz est utilisé pour isoler des signaux hémodynamiques du signal électrique traité V₄.

A partir du signal électrique traité, le module d'analyse 18 est configuré pour en extraire une variation de volume et/ou une variation de pression en fonction du temps proportionnelle(s) à une baisse de tension entre le dipôle émetteur 12 et le dipôle récepteur 14.

La **Figure 2** illustre schématiquement la propagation d'un signal électrique V₀ depuis le dipôle émetteur 12 formé par la paire d'électrodes E1, E2 jusqu'au dipôle récepteur 14 formé par la paire d'électrodes E3, E4 du système médical implantable 10.

Les éléments avec les mêmes références numériques déjà utilisées pour la description de la Figure 1 ne seront pas décrits à nouveau en détail, et référence est faite à leurs descriptions ci-dessus.

Dans l'état implanté du dispositif et par application du signal Vo, le dipôle émetteur 12 est utilisé pour créer un champ électrique E se propageant à travers des tissus d'un corps humain jusqu'au dipôle récepteur 14. Le dipôle récepteur 14 détecte une différence de potentiel qui dépend du champ électrique E, illustrée à la Figure 2 par le signal électrique V₁. Le signal électrique V₁ détecté dépend principalement de quatre facteurs qui sont : la longueur « d » du canal de propagation, c'est-à-dire la distance entre le dipôle émetteur 12 et le dipôle récepteur 14 ; l'orientation « α » des dipôles 12, 14 l'un par rapport à l'autre ; les distances inter-électrodes « dₑ₁ » et « dₑ₂ » des dipôles 12, 14, c'est-à-dire la distance entre les électrodes E1, E2 et la distance entre les électrodes E3, E4 ; et les propriétés électriques du milieu de propagation.

Comme le montre la Figure 2, les électrodes E3, E4 forment un dipôle récepteur dont l'orientation est différente du dipôle récepteur formé par les électrodes E3, E4'. La différence d'orientation entre les dipôles E3, E4 et E3, E4' est illustré par l'angle α à la Figure 2. Lorsque que le système médical implantable 10 est implanté dans un corps humain, notamment dans ou au voisinage du coeur, le signal électrique V₁ du dipôle récepteur 14 est modulé en amplitude. Cela résulte du fait que la respiration change les propriétés de l'environnement, notamment la quantité d'oxygène présente dans les poumons, ce qui fait varier l'atténuation du signal électrique au cours de sa transmission le long du canal de propagation et entraîne ainsi une variation de l'amplitude du signal électrique V₁.

La **Figure 3** représente, selon un deuxième exemple, l'intégration du système médical implantable 10 dans un dispositif implanté 100, ici un défibrillateur sous-cutané implantable. Dans une variante, le dispositif 100 est un enregistreur d'événement, par exemple un enregistreur en boucle implantable.

Le dispositif implantable sous-cutané 100 tel que représenté à la Figure 3 comprend un boîtier 102, trois électrodes 104, 106, 108 et une électrode de défibrillation 110. Le boîtier 102 du dispositif implantable sous-cutané 100 peut comprendre un module de télémétrie (non représenté).

Le dispositif implantable sous-cutané 100 est adapté pour intégrer le système médical implantable 10 selon la présente invention car il comprend au moins un dipôle émetteur et un dipôle récepteur dont les électrodes de chaque dipôle sont distinctes les unes des autres. Le tableau 1 ci-dessous liste des configurations de dipôles émetteurs et récepteurs qui peuvent être utilisées dans le dispositif implantable sous-cutané 100 pour mettre en oeuvre le système 10 selon la présente invention.

**[Tableau 1]**

| # | Dipôle émetteur | Dipôle récepteur |
|---|---|---|
| 1 | 104-106 | 108-102 |
| 2 | 104-108 | 106-102 |
| 5 | 110-106 | 108-102 |
| 6 | 110-108 | 106-102 |
| 7 | 104-102 | 106-108 |
| 8 | 104-102 | 110-106 |
| 9 | 104-102 | 110-108 |
| 10 | 110-102 | 106-108 |
| 11 | 110-102 | 104-106 |
| 12 | 110-102 | 104-108 |
| 13 | 108-102 | 104-106 |
| 14 | 106-102 | 104-108 |
| 15 | 108-102 | 110-106 |
| 16 | 106-102 | 110-108 |
| 17 | 106-108 | 104-102 |
| 18 | 110-106 | 104-102 |
| 19 | 108-102 | 104-102 |
| 20 | 106-108 | 110-102 |
| 21 | 104-106 | 110-102 |
| 22 | 104-108 | 110-102 |

Tel qu'indiqué dans le Tableau 1, l'une des électrodes peut être constituée par le boîtier 102 du dispositif 100. Toutes les combinaisons d'électrodes peuvent être utilisées, y compris l'électrode de défibrillation 110.

Le dispositif implantable sous-cutané 100 peut en outre comprendre un moyen de détection, formé par au moins un couple d'électrodes, parmi ceux listés dans le Tableau 1, configuré pour réaliser un électrocardiogramme sous-cutané à partir duquel peut être détecté un complexe PQRST. Ainsi, les ondes R et Q, en particulier, sont identifiables à partir du complexe PQRST.

Ainsi, le système 10 peut être mis en oeuvre au moyen d'un dispositif préexistant. De plus, un praticien peut avantageusement sélectionner la configuration de dipôles émetteur et récepteur la plus adaptée aux paramètres physiologiques qu'il souhaite recueillir. Dans une variante, le système 10 comprend un accéléromètre intégré dans le dispositif implantable sous-cutané 100, qui permet de détecter une activité mécanique du coeur. L'activité mécanique du coeur peut être comparée au signal électrique recueilli afin d'établir une corrélation entre les signaux.

La **Figure 4** représente l'intégration du système médical implantable 10 selon la présente invention dans un système multi-dispositifs 100, 200. Le système multi-dispositifs 100, 200 représenté à la Figure 4 comprend un dispositif implantable sous-cutané 100 selon le deuxième mode de réalisation et un dispositif intracardiaque, tel qu'un stimulateur cardiaque, en particulier un stimulateur sans sonde 200. Les éléments avec les mêmes références numériques déjà utilisées pour la description de la

Figure 3 ne seront pas décrits à nouveau en détail, et référence est faite à leurs descriptions ci-dessus.

Dans une variante, un enregistreur d'événement ou un enregistreur implantable en boucle pourrait être utilisé à la place du dispositif implantable sous-cutané 100. Un tel enregistreur d'événement ou enregistreur implantable en boucle peut comprendre un moyen de détection, formé par au moins un couple d'électrodes dudit enregistreur, et configuré pour réaliser un électrocardiogramme sous-cutané à partir duquel peut être détecté un complexe PQRST. Ainsi, les ondes R et Q, en particulier, sont identifiables à partir du complexe PQRST.

Le stimulateur sans sonde 200 comprend une électrode de pointe 202 disposée à une extrémité du stimulateur 200, une première électrode annulaire 204 et une deuxième électrode annulaire 206. Les électrodes 202, 204 ou 202, 206 ou 204, 206 peuvent former un dipôle récepteur ou un dipôle émetteur. Le stimulateur sans sonde 200 peut comprendre un module de télémétrie (non représenté).

Le stimulateur sans sonde 200 peut en outre comprendre un moyen de détection, formé par un couple d'électrodes comprenant deux des électrodes 202, 204, 206 et configuré pour réaliser un électrogramme à partir duquel peut être détectée l'onde R.

Chacun du dispositif implantable sous-cutané 100 et du stimulateur cardiaque sans sonde 200 comprend des électrodes qui peuvent servir de dipôle récepteur et de dipôle émetteur. Ainsi, aussi bien le dispositif implantable sous-cutané 100 que le stimulateur cardiaque sans sonde 200 peuvent servir d'émetteur ou de récepteur dans le système 10 implantable de la présente invention.

L'implantation du dispositif implantable sous-cutané 100 et du stimulateur cardiaque sans sonde 200 telle qu'illustrée à la Figure 4 est adaptée pour une mesure trans-thoracique et permet de détecter les changements de volume d'une autre chambre du coeur que celle dans laquelle est implantée le stimulateur cardiaque sans sonde 200.

Par exemple, en utilisant le dispositif implantable sous-cutané 100 comme émetteur et le stimulateur cardiaque sans sonde 200 implanté dans le ventricule droit comme récepteur, l'information relative à la contraction de l'oreillette (« atrial kick » en anglais) peut être récupérée par le stimulateur cardiaque sans sonde 200, étant donné que l'activité mécanique de l'oreillette modifie à la fois la quantité de sang présent dans le ventricule droit et l'orientation du stimulateur cardiaque sans sonde 200. L'information relative à la contraction de l'oreillette peut être utilisée par le stimulateur cardiaque sans sonde 200 afin d'adapter la stimulation à l'activité normale de l'oreillette.

De plus, le système 10 ayant au moins quatre électrodes, de sorte que le dipôle émetteur soit distinct du dipôle récepteur, il est possible d'obtenir une mesure d'impédance plus globale et donc davantage représentative du milieu environnant, qu'une mesure entre seulement deux électrodes d'une même sonde.

En outre, les deux dispositifs 100, 200 sont configurés pour intégrer les dipôles émetteur et récepteur : chacun des dispositifs 100, 200 peut ainsi aussi bien servir d'émetteur que de récepteur, selon les besoins du praticien. Ainsi, il est possible de sélectionner la configuration des dipôles la plus sensible et/ou la plus économe en énergie, notamment au cours de la vie du patient dans lequel les dispositifs 100, 200 sont implantés. Cette sélection peut être effectuée en temps réel au moyen d'un module de télémétrie.

Dans une variante, le système médical implantable de la présente invention peut être intégré dans un système multi-dispositifs comprenant un dispositif implantable sous-cutané, tel que le dispositif 100, et deux stimulateurs sans sonde, chacun étant du type du stimulateur 200, (l'un prévu pour être implanté dans le ventricule droit et l'autre dans l'oreillette droite), chacun du dispositif implantable sous-cutané et des stimulateurs sans sonde comprenant au moins un dipôle d'électrode émetteur et/ou récepteur. Un tel système est adapté pour une mesure trans-thoracique et permet de détecter les changements de volume observés dans le ventricule droit et dans l'oreillette droite. Un tel système permet ainsi d'avoir aussi une vision plus exhaustive de la mesure trans-thoracique. En outre, un des deux stimulateurs sans sonde est adapté pour stimuler le coeur dans l'oreillette droite. Dans une alternative à cette variante, l'un des deux stimulateurs sans sonde est prévu pour être implanté dans le ventricule gauche, au lieu de l'oreillette droite. Le fait qu'un stimulateur soit implanté dans le ventricule gauche et l'autre dans le ventricule droit rend possible une resynchronisation inter-ventriculaire.

Dans une autre variante, le système médical implantable de la présente invention peut être intégré dans un système multi-dispositifs comprenant un dispositif implantable sous-cutané, tel que le dispositif 100, et trois stimulateurs sans sonde, chacun tel que le stimulateur 200, (l'un prévu pour être implanté dans le ventricule droit, un autre dans l'oreillette droite et un autre encore dans le ventricule gauche), chacun du dispositif implantable sous-cutané et des stimulateurs sans sonde comprenant au moins un dipôle d'électrode émetteur et/ou récepteur. Un tel système constitue un système de resynchronisation cardiaque implantable dit « triple chambre » (ventricule droit, oreillette droite et ventricule gauche) qui est adapté non seulement au diagnostic mais aussi au traitement de l'insuffisance cardiaque. En effet, un système de resynchronisation cardiaque implantable a besoin d'un stimulateur sans sonde pour la stimulation dans le ventricule gauche afin de synchroniser la contraction intra-ventriculaire et interventriculaire.

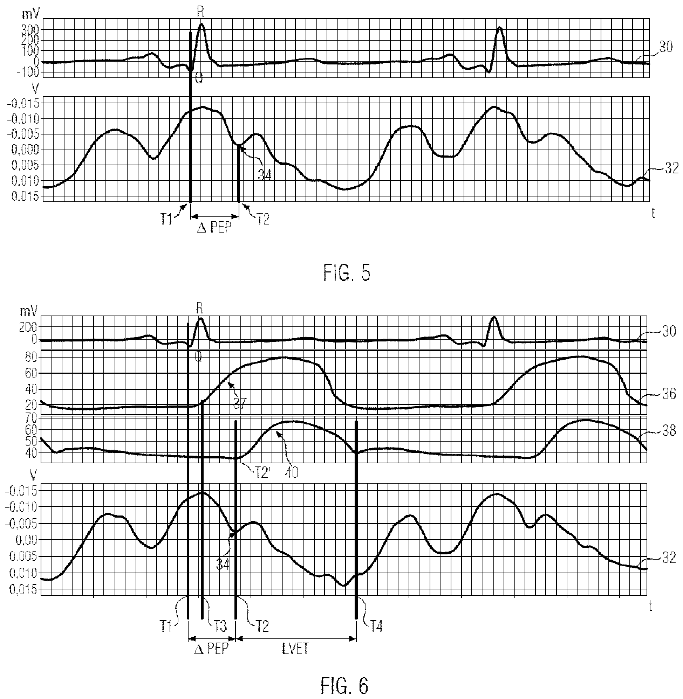

La **Figure 5** illustre un électrocardiogramme 30 et un signal électrique traité 32.

L'électrocardiogramme 30 est recueilli par le moyen 16 et le signal électrique 32 est recueilli et traité par le module d'analyse 18 du système 10 intégré dans le système multi-dispositifs 100, 200 tel que représenté à la Figure 4.

Les éléments avec les mêmes références numériques déjà utilisées pour la description des Figure 1 à 4 ne seront pas décrits à nouveau en détail, et référence est faite à leurs descriptions ci-dessus.

Le module d'analyse 18, en particulier le processeur 25, est configuré pour associer le signal électrique traité 32 et l'électrocardiogramme 30 pour en déterminer un paramètre représentatif d'une période pré-éjectionnelle.

La période pré-éjectionnelle correspond en hémodynamique à la contraction isovolumétrique qui précède l'éjection systolique. Elle est sous la dépendance du système nerveux sympathique et reflète la contractilité myocardique.

Une période pré-éjectionnelle est définie comme la durée entre le début du complexe QRS, c'est-à-dire d'une onde Q ou R, et l'ouverture de la valve aortique.

L'électrocardiogramme, indiqué par la référence 30 sur la Figure 5, permet d'identifier le pic de l'onde Q à partir du pic R du complexe PQRST. En effet, le pic R est plus facilement détectable que le pic de l'onde Q car le pic R a une plus grande amplitude que le pic Q.

Ainsi, l'identification du pic Q, indiquée par la référence T1 sur la Figure 5, permet de déterminer le début de la période pré-éjectionnelle.

Dans une variante, la détection de l'onde R est utilisée en tant que telle comme indicatrice du temps de début de la période pré-éjectionnelle ce qui permet de réduire la complexité du module d'analyse 18 du système 10 nécessaire à l'identification de l'onde Q.

Dans une autre variante, la détection de l'onde R est réalisée à partir d'un électrogramme.

L'ouverture de la valve aortique est déterminée au moyen du signal électrique traité 32 qui a été traité par le module d'analyse 18 du système 10. Le signal électrique traité 32 a été numérisé au moyen du convertisseur analogique-numérique 24 du système 10 et filtré numériquement afin de discriminer les informations respiratoires des informations hémodynamiques comprises dans le signal électrique. Un filtre bande passante de 0,5Hz à 30Hz est utilisé pour récupérer les informations hémodynamiques tandis qu'un filtre passe-bas, de préférence avec une fréquence de coupure f_{c} comprise entre 0,5Hz et 5Hz, en particulier f_{c}=1Hz, est utilisé pour récupérer les informations respiratoires. La plage de fréquences de 0,5Hz à 30Hz du filtre bande passante permet à la fois de filtrer l'artefact respiratoire en coupant les fréquences au-dessous de 0,5Hz et de filtrer les bruits à haute fréquence, c'est-à-dire les bruits dont la fréquence est au-dessus de 30Hz.

Dans une variante, lorsqu'un ajustement des fréquences est nécessaire, les valeurs des fréquences pour les filtres bande passante et passe-bas sont ajustées au moyen du processeur 25.

L'homme de l'art comprend que les informations respiratoires sont relatives à un changement de volume des poumons entraînant une modification de la propagation du champ électrique qui est à son tour détecté par le dipôle récepteur.

Tel qu'illustré à la Figure 5, il a été constaté que l'ouverture de la valve aortique indiquée par T2 correspond au premier minimum local 34 du signal électrique traité 32 suivant le début de l'onde Q, c'est-à-dire à partir de T1.

De ce fait, la durée entre le début de l'onde Q détectée au moyen de l'électrocardiogramme 30 et l'ouverture de la valve aortique détectée au moyen du signal électrique traité 32, c'est-à-dire la durée écoulée entre T1 et T2, permet de déterminer la période pré-éjectionnelle indiquée sur la Figure 6 par la référence ΔPEP. T2 correspond ainsi au temps de début de l'éjection.

La durée de la période pré-éjectionnelle ΔPEP peut servir d'indicateur lors de la surveillance d'une insuffisance cardiaque. Par exemple, plus la période pré-éjectionnelle ΔPEP est grande, moins le coeur est supposé fonctionner efficacement.

Le système 10 permet notamment de surmonter les difficultés rencontrées dans la détermination de la fin de la période pré-éjectionnelle au moyen de méthodes antérieures connues de l'homme du métier.

Dans une variante, le module d'analyse 18 est aussi apte, à partir du signal électrique traité 32, à surveiller un paramètre représentatif de l'efficacité d'une thérapie, tel que la fraction d'éjection ou le volume d'éjection, en tenant compte de l'évolution de la période pré-éjectionnelle ΔPEP mesurée sur une longue période, notamment une période de plusieurs semaines, mois ou années. Pour se faire, les valeurs de périodes pré-éjectionnelles ΔPEP mesurées peuvent être transférées au moyen d'un module de télémétrie du système 10 à un dispositif externe. Dans une variante, les valeurs de périodes pré-éjectionnelles ΔPEP mesurées peuvent être enregistrées sur un moyen de stockage, par exemple un moyen de stockage d'un des dispositifs 100, 200 du système 10.

Dans une autre variante, le système 10 est apte à détecter une activité mécanique du coeur à partir d'un signal acoustique représentatif des bruits du coeur. Le module d'analyse 18 est alors configuré pour comparer ledit signal acoustique au signal électrique traité. Dans encore une autre variante, le système 10 comprend un accéléromètre, par exemple intégré dans le dispositif implantable sous-cutané 100, qui permet de détecter une activité mécanique du coeur.

De ce fait, le système 10 peut être configuré pour détecter et recueillir l'activité mécanique/acoustique du coeur. Les informations ainsi recueillies relatives à l'activité mécanique/acoustique peuvent être utilisées pour corréler le signal électrique 32 recueilli par le module d'analyse 18 du système 10.

La **Figure 6** représente la comparaison entre des variations de pression aortique et ventriculaire gauche et le signal électrique recueilli et traité 32 représenté à la Figure 5.

Les éléments avec les mêmes références numériques déjà utilisées pour la description des Figure 1 à 5 ne seront pas décrits à nouveau en détail, et référence est faite à leurs descriptions ci-dessus.

Les variations de pression ventriculaire gauche, représentées par la courbe 36 de la Figure 6, et les variations de pression aortique, représentées par la courbe 38 de la Figure 6, ont été acquises à l'aide de deux cathéters de pression Millar intraventriculaires placés dans l'aorte et dans la ventriculaire gauche, selon une méthode connue de l'homme de l'art.

Sur la Figure 6, T3 indique le début d'une contraction du ventricule. En effet, tel que le montre la référence 37 sur la courbe 36 de la Figure 6, à partir de T3, la pression du ventricule gauche augmente.

Tel que le montre la référence 40 sur la courbe 38 de la Figure 6, à partir de T2', la pression aortique augmente lorsque le sang circule dans l'artère.

Après la dépolarisation des ventricules représentée par le complexe QRS de l'électrocardiogramme 30, le muscle cardiaque se contracte en augmentant la pression du ventricule gauche comme on peut le voir au temps T4 sur la courbe 36. La pression augmente jusqu'à atteindre une valeur suffisante pour ouvrir la valve aortique au temps T2'. Le temps compris entre T4 et T2' représente la durée de la contraction isovolumétrique du coeur qui est appelée ainsi car le volume sanguin dans les cavités ne change pas (les valves ne sont pas encore ouvertes). A T2', la valve aortique s'ouvre, permettant au sang de circuler à travers l'aorte, augmentant ainsi la pression de l'artère comme on peut le voir sur la courbe 38 de la Figure 6 indiquée par le numéro de référence 40.

La variation de la pression aortique 38 a ainsi été utilisée pour mesurer le temps T2' correspondant au début d'une éjection.

Tel qu'illustré par la Figure 6, le signal électrique traité 32 selon l'invention a un minimum local 34 qui correspond essentiellement au début de l'augmentation de la pression aortique 40 à T2' de la courbe 38.

La Figure 6 montre que T2' = T2, c'est-à-dire que l'abscisse du minimum local 34 observé sur le signal électrique traité 32 peut être attribuée au temps de l'ouverture de la valve aortique.

La variation de la pression aortique 38 peut aussi être utilisée pour déterminer la durée d'éjection ventriculaire gauche, indiquée sur la Figure 6 par la référence LVET pour « left ventricular éjection time » en anglais, qui correspond à la durée entre T2' et T4.

La **Figure 7** représente la comparaison entre la variation de volume du ventricule gauche déterminée à partir d'une mesure sonométrique et un signal électrique recueilli et traité par le module d'analyse 18 du système 10 intégré dans le système multi-dispositifs 100, 200 tel que représenté à la Figure 4.

Les éléments avec les mêmes références numériques déjà utilisées pour la description des Figure 1 à 6 ne seront pas décrits à nouveau en détail, et référence est faite à leurs descriptions ci-dessus.

La variation du volume ventriculaire gauche, indiquée par la courbe 42 à la Figure 7, a été déterminée au moyen d'une mesure sonométrique selon une méthode connue de l'homme de l'art.

La comparaison des courbes 32 et 42 de la Figure 7 montre que les pics, notamment les pics compris dans les intervalles 44 et 46, ainsi que la période des signaux, en particulier les périodes pré-éjectionnelles ΔPEP₁ et ΔPEP₂, sont préservées d'une courbe à l'autre. De plus, les courbes 32 et 42 de la Figure 7 mettent en évidence la répétition de motifs, tels que la répétition des intervalles 44 et 46. De ce fait, le signal électrique traité 32 a une variation similaire à celle du volume du ventricule gauche représenté par la courbe 42, illustrant ainsi la corrélation entre le signal électrique traité et les propriétés hémodynamiques du coeur.

La **Figure 8** illustre un électrocardiogramme 48, un phonocardiogramme 50, un signal électrique 52, un signal respiratoire 54 et un signal hémodynamique 56.

Les éléments avec les mêmes références numériques déjà utilisées pour la description des Figure 1 à 7 ne seront pas décrits à nouveau en détail, et référence est faite à leurs descriptions ci-dessus.

Le signal électrique 52 est un signal électrique recueilli par le module d'analyse 18 du système 10 intégré dans le dispositif implantable sous-cutanée 100 tel que représenté à la Figure 3.

Le signal électrique 52 représenté à la Figure 8 est un signal brut, c'est-à-dire qu'il n'a pas encore été traité numériquement par un filtre prévu à cet effet, tel que décrit en référence à la Figure 1.

Le phonocardiogramme 54 représenté à la Figure 8, a été déterminé au moyen d'une mesure acoustique selon une méthode connue de l'homme de l'art.

Dans un mode de réalisation, le système 10 peut comprendre des moyens de mesure acoustique aptes à recueillir un phonocardiogramme. Dans une variante, le système 10 peut comprendre un accéléromètre. Ainsi, le système 10 peut être configuré pour détecter et recueillir l'activité mécanique/acoustique du coeur. Les informations ainsi recueillies relatives à l'activité mécanique/acoustique peuvent être utilisées pour corréler le signal électrique 52 recueilli par le module d'analyse 18 du système 10.

Le signal respiratoire 54 et le signal hémodynamique 56 ont été extraits et discriminés par traitement numérique à partir du signal électrique 52. En particulier, un filtre bande passante de 1Hz à 30Hz est utilisé pour récupérer les informations hémodynamiques tandis qu'un filtre passe-bas, en particulier avec une fréquence de coupure f_{c} comprise entre 0,5Hz et 5Hz, en particulier f_{c}=1Hz, est utilisé pour récupérer les informations respiratoires à partir du signal électrique 52.

Tel qu'expliqué en référence aux Figures 5 à 7, l'électrocardiogramme 48 permet de déterminer le début d'une période pré-éjectionnelle.

Le temps T1 de la Figure 8 correspond au temps de début d'une période pré-éjectionnelle déterminée à partir de la détection d'une onde Q, T1 correspondant au pic Q.

Le temps T1' de la Figure 8 correspond au temps de début d'une période pré-éjectionnelle déterminée à partir de la détection d'une onde R, T1' correspondant au pic R.

T1 et T1' peuvent ainsi chacun indiquer le début de la période pré-éjectionnelle.

La Figure 8 permet de comparer le signal hémodynamique 56 recueilli, traité et extrait à partir du système 100 avec le signal du phonocardiogramme 50 et d'illustrer ainsi la corrélation entre ces deux signaux 50, 52.

Tel qu'illustré à la Figure 8, il a été constaté que l'ouverture de la valve aortique indiquée par T2 correspond au deuxième maximum local 58 du signal hémodynamique 56 suivant le début de l'onde Q ou R, c'est-à-dire respectivement à partir de T1 ou T1'.

Ainsi, une période de pré-éjection ΔPEP déterminée à partir de l'onde Q de la Figure 8 correspond à ΔPEP=T2-T1.

Une période de pré-éjection ΔPEP' déterminée à partir de l'onde R de la Figure 8 correspond à ΔPEP'=T2-T1'.

La durée de la période pré-éjectionnelle ΔPEP peut ainsi être déterminée à partir du dispositif implantable sous-cutanée 100 et peut servir d'indicateur lors de la surveillance d'une insuffisance cardiaque.

Le présent système fournit ainsi un moyen simplifié pour récupérer des informations à la fois respiratoires et hémodynamiques afin de déterminer un paramètre représentatif d'une période pré-éjectionnelle. Le présent système permet ainsi de déterminer une période pré-éjectionnelle sans qu'il soit nécessaire d'avoir recours à des capteurs de sons cardiaques, à partir desquels la détection d'une période pré-éjectionnelle est délicate, notamment à cause du rapport signal/bruit élevé du signal obtenu.

La détermination de la période pré-éjectionnelle fournit un indicateur adapté au diagnostic et à la surveillance de l'insuffisance cardiaque. D'ailleurs, dans la variante où le système comprend au moins un module de télémétrie, un message d'alerte peut être transmis au personnel soignant lorsque le module d'analyse détecte une augmentation anormale de la durée de la période pré-éjectionnelle au cours du temps, ce qui pourrait indiquer une insuffisance cardiaque.

Le présent système est ainsi adapté pour évaluer et optimiser une thérapie de resynchronisation cardiaque, où l'objectif est de réduire la période pré-éjectionnelle sans modifier la durée d'éjection ventriculaire gauche (appelé « left ventricular éjection time » en anglais).

## Revendications

1. Système médical implantable pour mesurer au moins un paramètre physiologique comprenant :
un premier dispositif médical implantable (100, 200) pourvu d'un dipôle émetteur (12), le dipôle émetteur (12) étant formé de deux électrodes (E1, E2) connectées à un générateur (16) et configuré pour émettre un signal électrique, et
un deuxième dispositif médical implantable (100, 200) distinct du premier dispositif médical implantable (100, 200) et pourvu d'un dipôle récepteur (14), le dipôle récepteur (14) étant formé de deux électrodes (E3, E4), chacune distincte des électrodes (E1, E2) du dipôle émetteur (12), le dipôle récepteur (14) étant configuré pour recueillir le signal électrique émis au moyen du dipôle émetteur (12),
un module d'analyse (18) comprenant au moins un amplificateur (20), un détecteur d'enveloppe (22) et un convertisseur analogique-numérique (24) et un moyen de traitement (25) pour traiter le signal électrique recueilli au moyen du dipôle récepteur (14);
un moyen de détection (26) configuré pour recueillir un électrocardiogramme ou un électrogramme ;
le module d'analyse (18) étant en outre configuré pour associer le signal électrique traité et l'électrocardiogramme ou l'électrogramme pour en déterminer un paramètre représentatif d'une période pré-éjectionnelle.

2. Le système selon la revendication 1, dont le module d'analyse (18) est configuré pour extraire du signal électrique traité une variation de volume et/ou une variation de pression en fonction du temps proportionnelle(s) à une baisse de tension entre le dipôle émetteur (12) et le dipôle récepteur (14).

3. Le système selon l'une des revendications précédentes, dans lequel la détermination de la période pré-éjectionnelle comprend la détection de l'onde R ou de l'onde Q d'un complexe QRS recueilli à partir du moyen de détection (26).

4. Le système selon l'une des revendications précédentes, dans lequel le module d'analyse (18) est apte à surveiller en outre un paramètre représentatif de l'efficacité d'une thérapie en tenant compte du paramètre représentatif d'une période pré-éjectionnelle.

5. Le système selon l'une des revendications précédentes, dans lequel les deux électrodes (E3, E4) du dipôle récepteur (14) sont configurées pour recueillir simultanément le signal électrique émis et un électrocardiogramme ou un électrogramme.

6. Le système selon l'une des revendications 1 à 4, dans lequel l'activation du module d'analyse (18) est déclenchée par la détection d'au moins un pic d'un complexe PQRST recueilli à partir du moyen de détection (26).

7. Le système selon l'une des revendications précédentes, comprenant un moyen apte à détecter une activité mécanique du coeur à partir d'un signal acoustique représentatif des bruits du coeur et dans lequel le module d'analyse est configuré pour comparer ledit signal acoustique au signal électrique traité.

8. Le système selon la revendication 1, dans lequel l'un du premier dispositif médical implantable ou du deuxième dispositif médical implantable (100, 200) est un défibrillateur automatique implantable sous-cutané ou un enregistreur d'événement ; et
l'autre du premier dispositif médical implantable ou du deuxième dispositif médical implantable (100, 200) est un dispositif endocavitaire implantable.

9. Le système selon la revendication 8, dans lequel le dispositif endocavitaire implantable (100, 200) est un stimulateur cardiaque sans sonde.

## Patentansprüche

1. Implantierbares medizinisches System zur Messung mindestens eines physiologischen Parameters, umfassend:
eine erste implantierbare medizinische Vorrichtung (100, 200), die mit einem Senderdipol (12) versehen ist, wobei der Senderdipol (12) aus zwei Elektroden (E1, E2) gebildet ist, die mit einem Generator (16) verbunden und zur Übertragung eines elektrischen Signals ausgestaltet sind, und
eine zweite implantierbare medizinische Vorrichtung (100, 200), die von der ersten implantierbaren medizinischen Vorrichtung (100, 200) verschieden ist und mit einem Empfängerdipol (14) versehen ist, wobei der Empfängerdipol (14) aus zwei Elektroden (E3, E4) gebildet ist, von denen jede von den Elektroden (E1, E2) des Senderdipols (12) verschieden ist, wobei der Empfängerdipol (14) so ausgestaltet ist, dass er das mittels des Senderdipols (12) übertragene elektrische Signal sammelt;
ein Analysemodul (18) mit mindestens einem Verstärker (20), einem Hüllkurvendetektor (22), einem Analog-Digital-Wandler (24) und einer Verarbeitungseinrichtung (25) zur Verarbeitung des mittels des Empfängerdipols (14) erfassten elektrischen Signals;
eine Detektionseinrichtung (26), die zur Erfassung eines Elektrokardiogramms oder eines Elektrogramms ausgestaltet ist,
wobei das Analysemodul (18) ferner so ausgestaltet ist, dass es das verarbeitete elektrische Signal mit dem Elektrokardiogramm oder dem Elektrogramm verknüpft, um daraus einen für eine Präejektionsperiode repräsentativen Parameter zu bestimmen.

2. System nach Anspruch 1, wobei das Analysemodul (18) so ausgestaltet ist, dass es aus dem verarbeiteten elektrischen Signal eine Volumenänderung und/oder eine Druckänderung über der Zeit extrahiert, die zu einem Spannungsabfall zwischen dem Senderdipol (12) und dem Empfängerdipol (14) proportional ist.

3. System nach einem der vorhergehenden Ansprüche, wobei die Bestimmung der Präejektionsperiode das Erfassen einer R-Welle oder einer Q-Welle eines von der Detektionseinrichtung (26) gesammelten QRS-Komplexes umfasst.

4. System nach einem der vorhergehenden Ansprüche, wobei das Analysemodul (18) dazu ausgestaltet ist, auch einen für die Wirksamkeit einer Therapie repräsentativen Parameter unter Berücksichtigung des für eine Präejektionsperiode repräsentativen Parameters zu überwachen.

5. System nach einem der vorhergehenden Ansprüche, wobei die beiden Elektroden (E3, E4) des Empfängerdipols (14) so ausgestaltet sind, dass sie zeitgleich das übertragene elektrische Signal und ein Elektrokardiogramm oder ein Elektrogramm erfassen.

6. System nach einem der Ansprüche 1 bis 4, wobei die Aktivierung des Analysemoduls (18) durch Erfassen mindestens eines Peaks eines von der Detektionseinrichtung (26) gesammelten PQRST-Komplexes ausgelöst ist.

7. System nach einem der vorhergehenden Ansprüche, umfassend eine Einrichtung, die dazu ausgestaltet ist, eine mechanische Aktivität des Herzens aus einem für Herztöne repräsentativen akustischen Signal zu erfassen und wobei das Analysemodul so konfiguriert ist, dass es das akustische Signal mit dem verarbeiteten elektrischen Signal vergleicht.

8. System nach Anspruch 1, wobei eine der ersten und zweiten implantierbaren medizinischen Vorrichtungen (100, 200) ein subkutan implantierbarer automatischer Defibrillator oder ein Ereignisrekorder ist; und die andere der ersten und zweiten implantierbaren medizinischen Vorrichtungen (100, 200) eine implantierbare Endokavitärvorrichtung ist.

9. System nach Anspruch 8, wobei die implantierbare Endokavitärvorrichtung (100, 200) ein kabelloser Herzschrittmacher ist.

## Claims

1. An implantable medical system for measuring at least one physiological parameter comprising:
a first implantable medical device (100, 200) provided with a transmitter dipole (12), the transmitter dipole (12) being formed of two electrodes (E1, E2) connected to a generator (16) and being configured to transmit an electrical signal, and
a second implantable medical device (100, 200) distinct from the first implantable medical device (100, 200) and provided with a receiver dipole (14), the receiver dipole (14) being formed of two electrodes (E3, E4), each one being distinct from the electrodes (E1, E2) of the transmitter dipole (12), the receiver dipole (14) being configured to collect the electrical signal transmitted by means of the transmitter dipole (12);
an analysis module (18) comprising at least an amplifier (20), an envelope detector (22), an analog-to-digital converter (24) and a processing means (25) for processing the electrical signal collected by means of the receiver dipole (14);
a detecting means (26) configured to collect an electrocardiogram or an electrogram,
the analysis module (18) being further configured to associate the processed electrical signal and the electrocardiogram or the electrogram to determine therefrom a parameter representative of a pre-ej ection period.

2. The system according to claim 1, wherein the analysis module (18) is configured to extract from the processed electrical signal a volume variation over time and/or a pressure variation over time that is/are proportional to a voltage drop between the transmitter dipole (12) and the receiver dipole (14).

3. The system according to any one of the preceding claims, wherein the determination of the pre-ejection period comprises detecting a R wave or a Q wave of a QRS complex collected from the detecting means (26).

4. The system according to any one of the preceding claims, wherein the analysis module (18) is adapted to further monitor a parameter representative of the efficiency of a therapy taking into account the parameter representative of a pre-ejection period.

5. The system according to any one of the preceding claims, wherein the two electrodes (E3, E4) of the receiver dipole (14) are configured to simultaneously collect the transmitted electrical signal and an electrocardiogram or an electrogram.

6. The system according to any one of claims 1 to 4, wherein the activation of the analysis module (18) is triggered by detecting at least one peak of a PQRST complex collected from the detecting means (26).

7. The system according to any one of the preceding claims, comprising a means adapted to detect a mechanical activity of the heart from an acoustic signal representative of heart sounds and wherein the analysis module is configured to compare said acoustic signal to the processed electrical signal.

8. The system according to claim 1, wherein:
one of the first and second implantable medical devices (100, 200) is a subcutaneous implantable automatic defibrillator or an event recorder; and
the other of the first and second implantable medical devices (100, 200) is an implantable endocardial device.

9. The system according to claim 8, wherein the implantable endocardial device (100, 200) is a leadless pacemaker.
